# EUROPEAN PATENT APPLICATION

(11) **EP 2 140 800 A1**
(43) Date of publication of application: **06.01.2010**
(21) Application number: 08740660.9
(22) Date of filing: 18.04.2008
(51) Int. Cl.: A61B 1/00, G06Q 50/00

(54) **MEDICAL INFORMATION MANAGEMENT NETWORK SYSTEM**

(30) Priority: 26.04.2007 JP 2007117588
(71) Applicant: Olympus Medical Systems Corp., Tokyo 151-0072 (JP)
(72) Inventor: ODA, Yasuharu, Tokyo 151-0072 (JP)
(74) Representative: von Hellfeld, Axel
(86) International application number: PCT/JP2008/057605
(87) International publication number: WO 2008/136283

(57) **Abstract**

An object of the present invention is to provide a medical-information management network system in which image display devices placed in a facility are used effectively. Workstations (4A to 4N) are connected with the medical-information management server (21) on a network (22) so as to construct a network system. An information manager (51a) included in the medical-information management server (21) collects equipment information, which is transferred from the workstations (4A to 4N), such as the number of pieces of used capsule endoscopes and the number of times a battery (35) for a receiving device (3) is used. Thus, the information manager 51a can unify the management of the available quantities or the usage statuses of the capsule endoscope and the battery (35) in the facility, and re-order timings can be predicted. Furthermore, pieces of unit-identification information of the capsule endoscope, the receiving device (3), and the workstations (4A to 4N), which are used for an examination of each subject, are collected by the medical-information management server (21) and managed in a unified way. Thus, the traceability of these equipments can be secured.

## Description

### TECHNICAL FIELD

The present invention relates to a medical-information management network system.

### BACKGROUND ART

In recent years, capsule endoscopes that have imaging and radio-communication functions have been developed in the field of endoscope. A capsule endoscope system has been proposed that uses such a capsule endoscope for capturing images of the inside of organs of a subject, sequentially receives the captured images with a receiving device, sequentially displays the internal images of the subject that are received by the receiving device on an image display device, such as a workstation, so that the inside of the subject can be observed (examined).

In the capsule endoscope system, patient information and examination information that are manually input into the image display device, such as a workstation, are transferred to the receiving device. The receiving device is detached from the image display device and attached to a body surface of the subject. Then, the receiving device stores as a maximum the number of images taken over an eight-hour period (about 60000 images) that are transmitted from a capsule endoscope that has been swallowed. After the capturing of images by the capsule endoscope, the receiving device is detached from the subject and then connected to the image display device again. When the receiving device is connected, the image display device receives the large amount of images that have been stored in the receiving device, and uses them for an image observation and a diagnosis report preparation.

Patent Document 1: Japanese Patent Application Laid-open No. 2006-61627

### DISCLOSURE OF INVENTION

### PROBLEM TO BE SOLVED BY THE INVENTION

The image display device such as a workstation is conventionally operated as a stand-alone apparatus (e.g., see Patent Document 1). Thus, when a plurality of workstations is placed in a facility such as a hospital, the workstations do not have a function for cooperating with each other. The workstations merely output diagnosis reports for respective diagnosis results obtained from capsule endoscope examinations. The workstations in facilities are not used effectively.

The present invention is made in view of the above, and an object of the present invention is to provide a medical-information management network system that can effectively use an image display device placed in a facility.

### MEANS FOR SOLVING PROBLEM

To solve the problem described above and achieve the object, a medical-information management network system according to the present invention includes: a receiving device that stores in-vivo images captured by a body-insertable apparatus that is inserted into a subject; one or more image display devices that are connected to the receiving device to obtain the in-vivo images stored in the receiving device and perform predetermined processes; and one or more medical-information management servers that are connected with the image display device on a network, wherein the medical-information management server has a management unit that collects information transferred from the image display device and unifies the management of the information.

In the medical-information management network system according to the present invention as set forth in the invention described above, the information transferred from the image display device has information on a number of pieces of the used body-insertable apparatus.

In the medical-information management network system according to the present invention as set forth in the invention described above, the information transferred from the image display device has unit-identification information on the body-insertable apparatus.

In the medical-information management network system according to the present invention as set forth in the invention described above, the information transferred from the image display device has information on a number of times a battery contained in the receiving device is used.

In the medical-information management network system according to the present invention as set forth in the invention described above, the information transferred from the image display device has information on a duration of use of a battery contained in the receiving device.

In the medical-information management network system according to the present invention as set forth in the invention described above, the information transferred from the image display device has unit-identification information on a battery contained in the receiving device.

In the medical-information management network system according to the present invention as set forth in the invention described above, the information transferred from the image display device has unit-identification information on the receiving device.

In the medical-information management network system according to the present invention as set forth in the invention described above, the information transferred from the image display device has pieces of unit-identification information on the image display device.

In the medical-information management network system according to the present invention as set forth in the invention described above, the information transferred from the image display device has a diagnosis result.

In the medical-information management network system according to the present invention as set forth in the invention described above, the diagnosis result has comments given based on an observation of in-vivo images obtained from the receiving device.

In the medical-information management network system according to the present invention as set forth in the invention described above, the information transferred from the image display device has an in-vivo image obtained from the receiving device.

In the medical-information management network system according to the present invention as set forth in the invention described above, the medical-information management server gives out, when the number of pieces of the used body-insertable apparatus reaches a predetermined number, a warning that an available quantity of body-insertable apparatuses is small.

In the medical-information management network system according to the present invention as set forth in the invention described above, the medical-information management server gives out, when the number of times the battery contained in the receiving device is used reaches a predetermined number, a notification that a life duration of the battery is about to expire.

In the medical-information management network system according to the present invention as set forth in the invention described above, the medical-information management server gives out, when the duration of use of the battery contained in the receiving device reaches a predetermined duration, a notification that a life duration of the battery is about to expire.

In the medical-information management network system according to the present invention as set forth in the invention described above, the medical-information management server uses unit-identification information on the receiving device so as to perform statistical processes on an operation rate of the receiving device.

In the medical-information management network system according to the present invention as set forth in the invention described above, the medical-information management server performs statistical processes on the diagnosis result.

### EFFECT OF THE INVENTION

In the medical-information management network system in accordance with the present invention, the image display device is connected with the medical-information management server on a network so as to construct a network system, and a management unit included in the medical-information management server collects information transferred from the image display device and unifies the management of the information. Thus, the image display device is used effectively, and pieces of information on the number of pieces of used body-insertable apparatuses in a facility and the number of times the battery for the receiving device is used are collected by the medical-information management server and managed in a unified way. Therefore, the available quantities of the body-insertable apparatuses and the batteries in a facility can be managed, and re-order timings of these equipments can be predicted based on the available quantities. Furthermore, pieces of unit-identification information, transferred from the image display device, of the body-insertable apparatus, the receiving device, and the image display device, which are used for the examination of each subject, are collected by the medical-information management server and managed in a unified way. Thus, the traceability of these equipments can be secured.

### BRIEF DESCRIPTION OF DRAWINGS

FIG. 1 is a schematic diagram showing an exemplary capsule endoscope system used in an embodiment of the present invention.
FIG. 2 is a schematic diagram showing an exemplary configuration of a medical-information management network system in accordance with the embodiment.
FIG. 3 is a block diagram showing an exemplary schematic configuration of the medical-information management network system in accordance with the embodiment.
FIG. 4 is an illustration of an exemplary capsule database.
FIG. 5 is an illustration of an exemplary battery database.
FIG. 6 is an illustration of an exemplary history database.

### EXPLANATIONS OF LETTERS OR NUMERALS

- 1: Subject
- 2: Capsule endoscope
- 3: Receiving device
- 4A to 4N: Workstation
- 21: Medical-information management server
- 22: Network
- 35: Battery
- 51a: Information manager
- 51b: Statistics manager

### BEST MODE(S) FOR CARRYING OUT THE INVENTION

Exemplary embodiments of a medical-information management network system in accordance with the present invention are described in detail with reference to accompanying drawings. Not limited to the embodiments, the present invention can be modified in various ways without departing from the spirit of the present invention; therefore, those embodiments do not limit the scope of the invention.

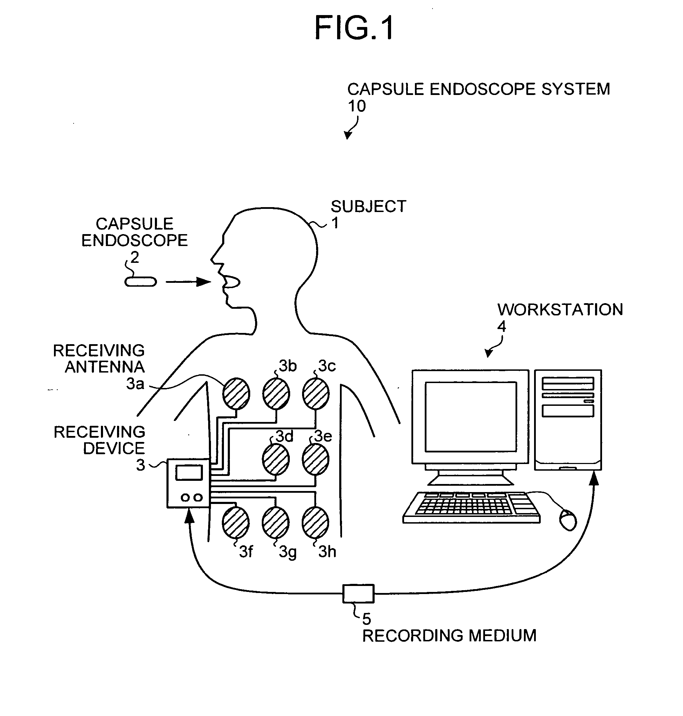

Prior to the description of the medical-information management network system of the present embodiment, an outline of a capsule endoscope system is described. FIG. 1 is a schematic diagram showing an exemplary capsule endoscope system. As shown in FIG. 1, a capsule endoscope system 10 includes a capsule endoscope 2 that captures in-vivo images of a subject 1, such as a patient, a receiving device 3 that receives the in-vivo images of the subject 1 from the capsule endoscope 2 and stores therein these images, a workstation 4 that obtains and displays in-vivo image information on the subject 1, which is received and stored by the receiving device 3, and performs other predetermined processes on the information, and a portable recording medium 5 for transferring data between the receiving device 3 and the workstation 4.

The capsule endoscope 2 captures in-vivo images of the subject 1. The capsule endoscope 2 has imaging and radio-communication functions in its capsule-shaped casing that is insertable into organs of the subject 1. Specifically, the capsule endoscope 2 is swallowed from the mouth of the subject 1. While moving through organs of the subject 1 due to peristalsis or the like of the organs, the capsule endoscope 2 sequentially captures in-vivo images of the subject 1 at predetermined intervals (e.g., 0.5-second intervals). The capsule endoscope 2 then sequentially and wirelessly transmits in-vivo information including in-vivo images of the subject 1 to the external receiving device 3. The capsule endoscope 2 has a capsule ID that identifies each individual capsule endoscope 2. The capsule ID is appended to image signals that include captured image data. Such a capsule endoscope 2 sequentially and wirelessly transmits the image data, such as in-vivo images, together with its capsule ID to the external receiving device 3.

The receiving device 3 wirelessly receives and stores therein the in-vivo images of the subject 1 that are captured by the capsule endoscope 2. In detail, the receiving device 3 includes a plurality of receiving antennas 3a to 3h and is attached to (carried by) the subject 1, who has the capsule endoscope 2 inserted into organs. The receiving device 3 sequentially receives the image signals that are wirelessly transmitted from the capsule endoscope 2 via the receiving antennas 3a to 3h and obtains the image data and capsule ID included in the image signal. Furthermore, the receiving device 3 includes the recording medium 5 and stores in the recording medium 5 the in-vivo images of the subject 1 and the capsule ID that are received from the capsule endoscope 2.

The workstation 4 obtains various types of data, such as in-vivo images of the subject 1, from the recording medium 5 and has a function as an image display device for displaying those obtained various types of data on a display. The in-vivo images of the subject 1 displayed by the workstation 4 are observed (examined) by users, such as doctors and nurses, and thus the subject 1 is diagnosed. Furthermore, the workstation 4 has a report generating function for generating a diagnosis report that shows a diagnosis result of the subject 1 and other information. The workstation 4 displays the generated diagnosis result on the display.

The recording medium 5 is a portable recording medium and is used for transferring data between the receiving device 3 and the workstation 4 described above. In detail, the recording medium 5 can be attached to and detached from the receiving device 3 or the workstation 4 and, when inserted into the receiving device 3 or the workstation 4, the recording medium 5 can output data or store data. When inserted into the receiving device 3, the recording medium 5 stores therein in-vivo images of the subject 1 and the like that are received by the receiving device 3 from the capsule endoscope 2. When inserted into the workstation 4, the recording medium 5 outputs the stored data, such as in-vivo images of the subject 1, to the workstation 4. In the present embodiment, the portable recording medium 5 is used for transferring data between the receiving device 3 and the workstation 4; therefore, the recording medium 5 is a component of the receiving device 3. Alternatively, the receiving device 3 may be directly connected to the workstation 4 so that data can be transferred.

The various types of data stored in the recording medium 5 are, for example, an in-vivo image group of the subject 1, time information (e.g., capture times and reception times) of each in-vivo image in the in-vivo image group, the capsule ID of the capsule endoscope 2 that has captured the in-vivo image group of the subject 1, patient information on the subject 1, and examination information on the subject 1. The patient information on the subject 1 is information for identifying the subject 1, e.g., patient name, patient ID, birth date, sex, and age. The examination information on the subject 1 is information for identifying a capsule endoscope examination (an examination in which the capsule endoscope 2 is inserted into organs in order to observe inside of the organs) that is performed on the subject 1, e.g., examination ID and examination date, which differ depending for each examination.

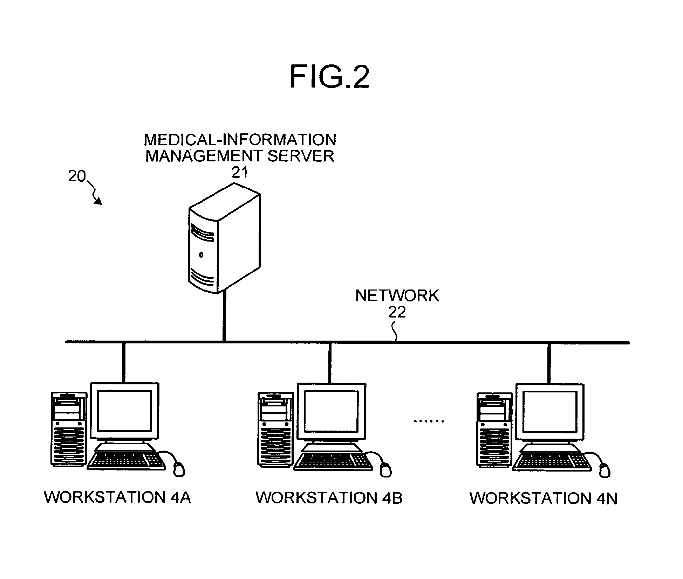

A medical-information management network system in accordance with the present embodiment that includes the receiving device 3 (including the recording medium 5) and the workstation 4 is described below. FIG. 2 is a schematic diagram showing an exemplary configuration of a medical-information management network system in accordance with the present embodiment. A medical-information management network system 20 includes a medical-information management server 21, a plurality of workstations 4A to 4N, a network 22, and the receiving device 3 described later. The medical-information management network system 20 is installed in a certain facility such as a certain hospital with the plurality of workstations 4A to 4N placed at certain locations. A plurality of the medical-information management server 21 may be included in the medical-information management network system 20, and the number of workstations may be only one.

The medical-information management server 21 unifies the management of various types of information that are transferred from the workstations 4A to 4N. The network 22 is constructed with the Internet, an intranet, a wired LAN, a wireless LAN, or the like.

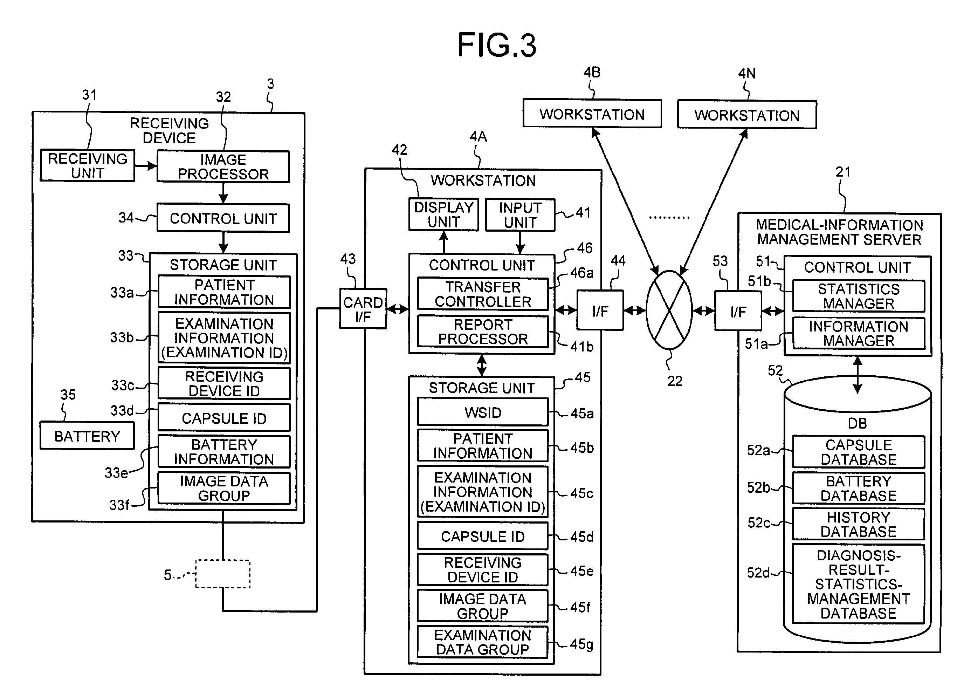

Exemplary configurations of the medical-information management server 21, the workstations 4A to 4N, and the receiving device 3, which are included in such a system, are described with reference to FIG. 3. FIG. 3 is a block diagram showing an exemplary configuration of the medical-information management network system 20.

The configuration of the receiving device 3 (the recording medium 5) is described first. The receiving device 3 includes a receiving unit 31 that receives image signals wirelessly transmitted from the capsule endoscope 2, an image processor 32 that generates in-vivo images of the subject 1 based on the image signals, a storage unit 33 into which the recording medium 5 described above is inserted, a control unit 34 that controls the components of the receiving device 3, and a battery 35 that supplies power to the components of the receiving device 3. The storage unit 33 that corresponds to the recording medium 5 includes storage areas 33a and 33b that store the patient information and the examination information (that includes the examination ID), respectively. The patient information and examination information are transferred from a workstation 4A before the examination. The receiving device 3 has a receiving device ID, which is unit-identification information that identifies the receiving device 3 itself. Thus, the storage unit 33 includes a storage area 33c that stores the receiving device ID. Furthermore, the storage unit 33 includes a storage area 33d that stores the capsule ID transmitted from the capsule endoscope 2. The storage unit 33 includes a storage area 33e that stores battery information such as the number of times the battery 35 installed in the receiving device 3 is used. The storage unit 33 includes a storage area 33f that stores the image group obtained from the capsule endoscope 2 as the examination is performed.

The configuration of the workstation 4A (as an example) is described below. The remaining workstations 4B-4N have the same configuration. The workstation 4A includes an input unit 41 that inputs the various types of information, a display unit 42 that displays in-vivo images of the subject 1, and a card interface (I/F) 43 into which the recording medium 5 is inserted. Furthermore, the workstation 4 includes an interface (I/F) 44 that allows the workstation 4 to communicate information with the medical-information management server 21 on the network 22, a storage unit 45 that stores various types of data such as in-vivo images of the subject 1, and a control unit 46 that controls the components of the workstation 4A.

The input unit 41 can be constructed with a use of input devices such as a keyboard, a mouse, or the like. The input unit 41 inputs various types of information to the control unit 46 based on input operations from users. The display unit 42 can be constructed with a use of various types of displays such as a liquid crystal display or the like. The display unit 42 displays various types of information that are to be displayed based on the instruction from the control unit 46. For example, the display unit 42 displays in-vivo images of the subject 1 that are captured by the capsule endoscope 2, diagnosis reports on the subject 1, or the like. The recording medium 5 is inserted into the card I/F 43 in a manner such that the recording medium 5 can be detached therefrom. Then, the card I/F 43 enables data to be input or output between the inserted recording medium 5 and the control unit 46. The interface 44 is connected, for communication, with the medical-information management server 21 on the network 22 so that information is communicated.

The storage unit 45 can be constructed with a use of various types of storage mediums, such as a RAM, EEPROM, flash memory, and hard disk, which can store data therein in a rewritable manner. The storage unit 45 stores various types of data that are to be stored based on instructions from the control unit 46. The storage unit 45 transfers data, in the various types of stored data, that is to be read based on instructions from the control unit 46 to the control unit 46. The storage unit 45 includes a storage area 45a that stores a WSID, which is a unit-identification information for identifying the workstation (WS) 4A itself, a storage area 45b that stores patient information that is input and set, and a storage area 45c that stores examination information that is input and set. Furthermore, the storage unit 45 includes storage areas 45d and 45e that store the capsule ID and the receiving device ID that are transmitted from the receiving device 3 (the recording medium 5), respectively, a storage area 45f that stores an image data group, and a storage area 45g that stores examination data group indicating symptoms of a plurality of subjects. An examination data on a subject included in the examination data group includes an in-vivo image group of the subject, patient information, examination information, time information of each in-vivo image, and diagnosis report information.

The control unit 46 controls the components (the input unit 41, the display unit 42, the card I/F 43, the interface 44, and the storage unit 45) of the workstation 4 and controls input and output of signals among these components. Furthermore, the control unit 46 reads the examination data of a desired subject from the examination data group based on instruction information that is input from the input unit 41. The control unit 46 then displays on the display unit 42 the in-vivo image group and the like of the subject included in the examination data being read. The control unit 46 of the present embodiment includes a transfer processor 46a and a report processor 46b. When a diagnosis result and the like are transferred to the medical-information management server 21 after the examination is finished, the transfer processor 46a performs a process to append the various types of information referring to the storage unit 45 and the receiving device 3 (the recording medium 5).

A piece of information appended by the transfer processor 46a is information on the number of pieces of used capsule endoscopes 2. The capsule endoscope 2 is disposed after each examination is performed on the subject 1; therefore, each capsule endoscope system 10 uses one or more capsule endoscopes. In this case, the capsule endoscope 2 being used for the examination transmits wirelessly information on its capsule ID, which is then received by the receiving device 3 so that the workstation 4A can receive, for each examination, the capsule ID of the capsule endoscope being used. Furthermore, there are several types of the capsule endoscope 2: for use in the large intestine, small intestine, and stomach. These types can be identified based on the capsule ID.

Also, another piece of information appended by the transfer processor 46a is information on the number of times the battery 35 contained in the receiving device 3 is used. As for the examination using the capsule endoscope 2, a single examination takes at least eight hours to finish. The battery 35 contained in the receiving device 3 deteriorates over the periods of time it is used. The battery 35 can be used for only a limited number of times; therefore, the battery 35 needs to be replaced by the time when the battery 35 may not be able to be used for eight successive hours. For example, supposing that the number of times the battery 35 can be used be as a maximum ten, a battery replacement for the battery 35 is ordered when the number of times the battery 35 is used reaches eight. The information on the number of times the battery 35 is used can be a battery ID that can identify the battery 35 being used for an examination. Such battery ID information is stored as the information on the number of times the battery is used in the storage area 33e, which is an area for the battery information, of the receiving device 3. The battery ID information can be obtained by the transfer processor 46a when the receiving device 3 is connected with the workstation 4A after the examination.

Furthermore, another piece of information appended by the transfer processor 46a is a capsule ID, a receiving device ID, or a WSID, which are pieces of unit-identification information for identifying the capsule endoscope 2, the receiving device 3, and the workstation 4A being used for an examination of a certain subject 1, respectively. These pieces of information can be obtained by the transfer processor 46a from the storage area 45a therein and the storage areas 33d and 33c when the receiving device 3 is connected with the workstation 4A after the examination.

The report processor 46b functions as a report generator that generates, based on the examination data of the subject selected from the examination data group, a diagnosis report on the subject as a diagnosis result. The report processor 46b generates a diagnosis report in a unified format that shows, for example, an in-vivo image group of the subject 1; a capsule ID of the capsule endoscope 2 that has captured the in-vivo image group of the subject 1; patient information such as patient name, sex, age, birth date, and patient ID of the subject 1; examination information such as examination date and examination ID of the subject 1; and a diagnosis result (i.e., disease name) of the subject 1 posted as comments given based on the observation.

Furthermore, a configuration of the medical-information management server 21 is described. The medical-information management server 21 includes a control unit 51, a database (DB) 52, and an interface (I/F) 53. The interface 53 enables the connection of the medical-information management server 21 with the network 22. The control unit 51 includes an information manager 51a and a statistics manager 51b.

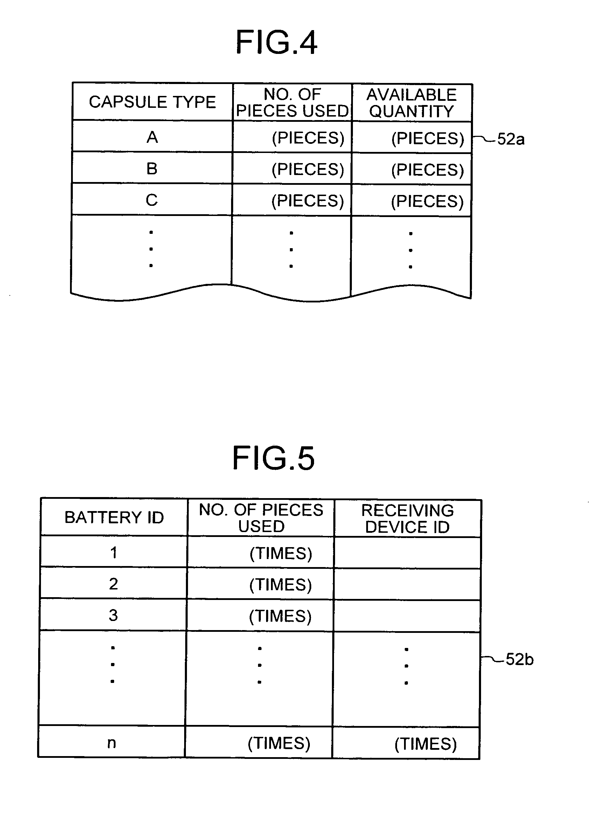

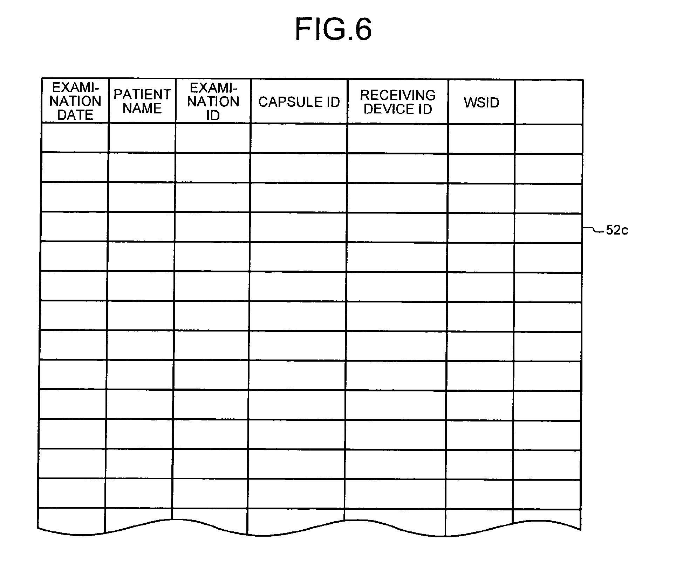

The information manager 51a collects various types of information that are transferred from the workstations 4A to 4N, especially information appended by the transfer processor 46a so that the information manager 51a unifies the management of the information. For example, based on the information on the number of pieces of used capsule endoscopes 2 that is transferred from the workstations 4A to 4N, the information manager 51a unifies the management on the available quantity of the capsule endoscopes 2 in a facility at a capsule database 52a. FIG. 4 is an illustration of an exemplary capsule database 52a. Furthermore, for example, based on the number of times the battery 35 is used that is transferred from the workstations 4A to 4N, the information manager 51a unifies the management on the usage status of the battery 35 in a facility at a battery database 52b. FIG. 5 is an illustration of an exemplary battery database 52b. Furthermore, for example, the information manager 51a refers to a capsule ID, a receiving device ID, and a WSID, which are pieces of unit-identification information that respectively correspond to the capsule endoscope 2, the receiving device 3, and the workstations 4A to 4N that are used for an examination of each subject 1, so that the information manager 51a can unify the management on an examination history at a history database 52c. FIG. 6 is an illustration of an exemplary history database 52c.

Furthermore, the report generator 51a performs processes to generate a diagnosis report for each subject 1 in a unified format based on the diagnosis result of each subject 1 that is transferred from the workstations 4A to 4N. The diagnosis report includes patient information such as patient name, sex, age, and birth date; examination information such as examination date and examination ID; a diagnosis result (disease name); and an extracted distinctive image used for a diagnosis.

The statistics manager 51b collects diagnosis results included in the diagnosis reports, which are transferred from the workstations 4A to 4N after the examination are made, in a unified format, so that the statistics manager 51b performs a statistics management on the diagnosis results. The statistics management is performed in a manner such that statistics of relations between a disease name included in the diagnosis result and a distinctive capsule image identifying the disease name are cumulatively taken and managed.

In such a system configuration, the patient information and the examination, which are manually input to the workstation 4A, are transferred to the receiving device 3 before the examination is performed. The receiving device 3 is detached from the workstation 4A and attached to the body surface of the subject 1. There, the receiving device 3 stores therein as a maximum the number of images taken over an eight-hour period (about 60000 images) that are transmitted from the capsule endoscope 2. After the examination using the capsule endoscope 2, the receiving device 3 is detached from the subject 1 and then connected to the workstation 4A again (i.e., the recording medium 5 is inserted). When the receiving device 3 is connected (i.e., when the recording medium 5 is inserted), the workstation 4A receives the large amount of images that have been stored in the receiving device 3, and thus the images can be observed and the diagnosis can be performed. The same applies to the remaining workstations 4B-4N.

When a diagnosis report on the subject 1, who has been examined, is generated after the examination, the workstation 4A transfers the diagnosis report to the medical-information management server 21. Receiving the transferred diagnosis report, the statistics manager 51b in the medical-information management server 21 statistically manages the relations between distinctive capsule image data and disease names that are included in the transferred diagnosis report in a manner such that these relations are accumulated and managed on a diagnosis-result-statistics-management database 52d. As a result, the examination data group in the storage area 45g can be updated and the diagnosis result can be more accurate.

When the workstations 4A to 4N transfer diagnosis reports after the examination, the transfer processor 46a in the workstations 4A to 4N appends the capsule ID information and the information on the number of pieces of used capsule endoscopes 2 to the diagnosis report and then transfers the diagnosis report to the medical-information management server 21. The information manager 51c identifies a capsule type based on the capsule ID information thus transferred. On the capsule database 52a shown in FIG. 4, the information manager 51c increments by one the number of pieces thus used of the identified capsule type by one and decrements by one the available quantity in a facility that is previously set.

As described, all workstations 4A to 4N in a facility are used effectively so that information on the number of pieces of used capsule endoscopes 2 is collected by the medical-information management server 21. Thus, the available quantity of the capsule endoscopes 2 in the facility can be managed in a unified way, and a re-order timing of the capsule endoscope 2 can be predicted based on the available quantity. Therefore, when the number of pieces of used capsule endoscopes 2 reaches a predetermined number, the medical-information management server 21 can warn users that the available quantity of the capsule endoscope 2 is small.

Furthermore, when the workstations 4A to 4N transfer diagnosis reports after the examination, the transfer processor 46a in the workstations 4A to 4N reads from the receiving device 3 (the recording medium 5) the battery ID information for identifying the battery 35 contained in the receiving device 3 and appends the battery ID information to the diagnosis report and then transfers the diagnosis report to the medical-information management server 21. As shown in FIG. 5, based on the battery ID information, the information manager 51c increments by one the number of times thus used field corresponding to the identified battery ID field on the battery database 52b.
At the same time, the information manager 51c updates the receiving device ID information on the battery database 52b so that the receiving device 3 containing the battery 35 can be identified.

As described, all the workstations 4A to 4N in a facility are used effectively so that the number of times each battery 35 is used is collected by the medical-information management server 21. Thus, a consumed state of the battery 35 in the facility can be managed in a unified way, and a re-order timing of the battery 35 to be replaced can be predicted based on the consumed state. For example, supposing that the number of times used be as a maximum ten, and the number of batteries 35 that are used eight times in a facility increases, it is predicted that the re-order timing of the battery 35 has come. The eight times is close to the maximum number of times used. Then, the user can be notified of information that the life duration of the battery 35 contained in the receiving device 3 is about to expire.

One of pieces of information appended by the transfer processor 46a may be the duration of use of the battery 35 contained in the receiving device 3, instead of or together with the information on the number of times the receiving device 3 is used. As for the examination using the capsule endoscope 2, a single examination takes at least eight hours to finish. The battery 35 contained in the receiving device 3 deteriorates over the periods of time it is used. Therefore, the battery 35 can be used for only a limited duration and needs to be replaced by the time when the battery 35 may not be able to be used for eight successive hours. For example, supposing that the maximum duration of use of the battery 35 be 80 hours, it is determined that the re-order timing of the battery 35 has come when the duration of use reaches 70 hours. The information on duration of use of the battery 35 can be accumulated by obtaining the battery ID for identifying the battery 35 used for the examination and the examination time from the transfer processor 46a.

The same described above applies to the duration of use of each battery 35; when the workstations 4A to 4N transfer diagnosis reports after the examination, the transfer processor 46a in each of the workstations 4A to 4N reads from the receiving device 3 (the recording medium 5) the battery ID information for identifying the battery 35 contained in the receiving device 3 and the examination time information and appends these pieces of information to the diagnosis report and then transfers the diagnosis report to the medical-information management server 21. Based on the battery ID information and the examination time, the information manager 51c accumulates the examination time on the duration of use field corresponding to the identified battery ID field on the battery database 52b. At the same time, the information manager 51c updates the receiving device ID information on the battery database 52b so that the receiving device 3 containing the battery 35 can be identified.

As described, all the workstations 4A to 4N in a facility are used effectively so that the duration of use of each battery 35 is collected by the medical-information management server 21. Thus, a consumed state of the battery 35 in the facility can be managed in a unified way, and a re-order timing of the battery 35 to be replaced can be predicted based on the consumed state. The medical-information management server 21 can predict the re-order timing of the battery 35 has come, when the duration of use of the battery 35 contained in the receiving device 3 reaches a predetermined duration. The medical-information management server 21 then can notify the user that the life duration of the battery 35 contained in the receiving device 3 is about to expire.

Furthermore, when the workstations 4A to 4N transfer diagnosis reports after the examination, the transfer processor 46a in each of the workstations 4A to 4N appends, to the diagnosis report, equipment information such as a capsule ID, a receiving device ID, and a WSID that respectively correspond to the capsule endoscope 2, the receiving device 3, and the workstations 4A used for the examination of each subject 1, and then transfers the diagnosis report to the medical-information management server 21. As shown in FIG. 6, the information manager 51c accumulates these transferred pieces of unit-identification information, the patient information and the examination information included in the diagnosis result on the history database 52c as the equipment history information for each examination. Therefore, the medical-information management server 21 can statistically manage, for example, an operation rate of the receiving device 3, based on the unit-identification information of the receiving device 3.

As described, all the workstations 4A to 4N in a facility are used effectively so that the pieces of unit-identification information that respectively correspond to the capsule endoscope 2, the receiving device 3, and the workstations 4A to 4N used for the examination of each subject 1 are collected in the history database 52c in the medical-information management server 21. Thus, the traceability of these equipments can be secured. Especially, because the capsule endoscope 2 is inserted into the subject 1 in the examination and excreted from the subject 1 after the examination to be disposed, it is difficult to trace after the examination the history, i.e., who used which capsule endoscope 2 for the examination. Since the history for each examination is associated with the capsule ID and managed in the history database 52c, the history, such as who used which capsule endoscope 2 for the examination, can be traced as needed after the examination. Therefore, the traceability can be secured. Thus, for example, when there is a problem in a capsule endoscope 2 used for an examination, subjects who have had examinations using capsule endoscopes that were produced at the same production lot as the capsule endoscope 2 can be identified.

### INDUSTRIAL APPLICABILITY

As described, the medical-information management network system in accordance with the present invention is useful for effectively using image display devices placed in a facility such as a hospital, and, in particular, suitable for the inventory management of capsule endoscopes, batteries for receiving devices, or the like in a facility.

## Claims

1. A medical-information management network system, comprising:
a receiving device that stores in-vivo images captured by a body-insertable apparatus that is inserted into a subject;
one or more image display devices that are connected to the receiving device to obtain the in-vivo images stored in the receiving device and perform predetermined processes; and
one or more medical-information management servers that are connected with the image display device on a network, wherein
the medical-information management server has a management unit that collects information transferred from the image display device and unifies the management of the information.

2. The medical-information management network system according to claim 1, wherein
the information transferred from the image display device comprises information on a number of pieces of the used body-insertable apparatus.

3. The medical-information management network system according to claim 1, wherein
the information transferred from the image display device comprises unit-identification information on the body-insertable apparatus.

4. The medical-information management network system according to claim 1, wherein
the information transferred from the image display device comprises information on a number of times a battery contained in the receiving device is used.

5. The medical-information management network system according to claim 1, wherein
the information transferred from the image display device comprises information on a duration of use of a battery contained in the receiving device.

6. The medical-information management network system according to claim 1, wherein
the information transferred from the image display device comprises unit-identification information on a battery contained in the receiving device.

7. The medical-information management network system according to claim 1, wherein
the information transferred from the image display device comprises unit-identification information on the receiving device.

8. The medical-information management network system according to claim 1, wherein
the information transferred from the image display device comprises pieces of unit-identification information on the image display device.

9. The medical-information management network system according to claim 1, wherein
the information transferred from the image display device comprises a diagnosis result.

10. The medical-information management network system according to claim 9, wherein
the diagnosis result comprises comments given based on an observation of in-vivo images obtained from the receiving device.

11. The medical-information management network system according to claim 1, wherein
the information transferred from the image display device comprises an in-vivo image obtained from the receiving device.

12. The medical-information management network system according to claim 2, wherein
the medical-information management server gives out, when the number of pieces of the used body-insertable apparatus reaches a predetermined number, a warning that an available quantity of body-insertable apparatuses is small.

13. The medical-information management network system according to claim 4, wherein
the medical-information management server gives out, when the number of times the battery contained in the receiving device is used reaches a predetermined number, a notification that a life duration of the battery is about to expire.

14. The medical-information management network system according to claim 5, wherein
the medical-information management server gives out, when the duration of use of the battery contained in the receiving device reaches a predetermined duration, a notification that a life duration of the battery is about to expire.

15. The medical-information management network system according to claim 7, wherein
the medical-information management server uses unit-identification information on the receiving device so as to perform statistical processes on an operation rate of the receiving device.

16. The medical-information management network system according to claim 9, wherein
the medical-information management server performs statistical processes on the diagnosis result.
